(19)

Europäisches
Patentamt

European
Patent Office

Office européen
des brevets

(11) **EP 3 294 776 B1**

(12) **EUROPEAN PATENT SPECIFICATION**

(45) Date of publication and mention
of the grant of the patent:
**05.08.2020 Bulletin 2020/32**

(21) Application number: **16726231.0**

(22) Date of filing: **10.05.2016**

(51) Int Cl.:
*C08B 13/00* $^{(2006.01)}$        *A61K 47/38* $^{(2006.01)}$

(86) International application number:
**PCT/US2016/031584**

(87) International publication number:
**WO 2016/186895 (24.11.2016 Gazette 2016/47)**

(54) **PROCESS OF PREPARING A HIGH MOLECULAR WEIGHT ESTERIFIED CELLULOSE ETHER**

HERSTELLUNG ESTERIFIZIERTER CELLULOSEETHER MIT HOHEM MOELKULARGEWICHT

PROCESSUS DE PRODUCTION DE CELLULOSE ETHERS ESTÉRIFIÉS DE POIDS MOLÉCULAIRE ÉLEVÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.05.2015 US 201562162097 P**

(43) Date of publication of application:
**21.03.2018 Bulletin 2018/12**

(73) Proprietor: **Dow Global Technologies LLC
Midland, MI 48674 (US)**

(72) Inventors:
• **PETERMANN, Oliver
29699 Bomlitz (DE)**
• **BRACKHAGEN, Meinolf
29699 Bomlitz (DE)**

(74) Representative: **f & e patent
Braunsberger Feld 29
51429 Bergisch Gladbach (DE)**

(56) References cited:
**EP-A1- 0 018 547        EP-A1- 2 810 660
EP-A2- 0 018 605        WO-A1-2011/159626
WO-A1-2014/031448      GB-A- 2 006 217
US-A1- 2014 249 235**

• **Wanwilai Darunkaisorn ET AL: "HPMC Matrix
Granule Formation: Selection of Suitable
Granulating Fluid", Thai Pharmaceutical and
Health Science Journal Thai Pharm Health Sci J,
vol. 44, no. 29, 1 January 2009 (2009-01-01), pages
29-45, XP055502947,**
• **Xuefei Cao ET AL: "Rapid Synthesis of Cellulose
Esters by Transesterification of Cellulose with
Vinyl Esters under the Catalysis of NaOH or KOH
in DMSO", Journal of Agricultural and Food
Chemistry, vol. 61, no. 10, 27 February 2013
(2013-02-27), pages 2489-2495, XP055503022, US
ISSN: 0021-8561, DOI: 10.1021/jf3055104**

**Description**

FIELD

The present invention relates to an improved process for preparing an esterified cellulose ether.

INTRODUCTION

Esters of cellulose ethers, their uses and processes for preparing them are generally known in the art. Known methods of producing cellulose ether-esters include the reaction of a cellulose ether with an aliphatic monocarboxylic acid anhydride or a di- or tricarboxylic acid anhydride or a combination thereof, for example as described in U.S Patent Nos. 4,226,981 and 4,365,060.

Various known esters of cellulose ethers are useful as enteric polymers for pharmaceutical dosage forms, such as methylcellulose phthalate, hydroxypropyl methylcellulose phthalate, methylcellulose succinate, or hydroxypropyl methylcellulose acetate succinate (HPMCAS). Enteric polymers are those that are resistant to dissolution in the acidic environment of the stomach. Dosage forms coated with such polymers protect the drug from inactivation or degradation in the acidic environment or prevent irritation of the stomach by the drug. US Patent No. 4,365,060 discloses enterosoluble capsules which are said to have excellent enterosolubility behavior.

European Patent Application EP 0 219 426 discloses a process for producing an enteric-soluble acidic dicarboxylic acid ester of a cellulose ether wherein (a) a cellulose ether having hydroxypropoxyl groups as the ether-forming groups, of which a 2% by weight aqueous solution has a viscosity of at least 5 centipoise at 20 °C, is reacted with (b) a dicarboxylic acid anhydride or a mixture thereof with an anhydride of an aliphatic monocarboxylic acid in the presence of (c) a combination of an alkali metal acetate and acetic acid. EP 0 219 426 shows that the acidic dicarboxylic acid esters produced from cellulose ethers which have a viscosity of at least 6 centipoise provided an enterosoluble film-coating material on tablets which had resistance against a simulated gastric juice. When comparative acidic dicarboxylic acid esters were produced from cellulose ethers having a viscosity of only 3 centipoise, a substantial number of tablets disintegrated in the simulated gastric juice. Acidic dicarboxylic acid esters produced from cellulose ethers of higher viscosity have a higher molecular weight than those produced from cellulose ethers of lower viscosity when comparable process and recipe parameters for producing the acidic dicarboxylic acid esters are applied.

It is known in the prior art, e.g. as published in International Patent Application WO 2005/115330, that HPMCAS is also useful to increase the bioavailability of poorly water-soluble drugs. This is of great importance as nearly 70% of new drug candidates are low water soluble compounds. As a general rule, poorly water soluble drugs possess low bioavailability. The HPMCAS is aimed at reducing the crystallinity of the drug, thereby minimizing the activation energy necessary for the dissolution of the drug, as well as establishing hydrophilic conditions around the drug molecules, thereby improving the solubility of the drug itself to increase its bioavailability, i.e., its *in vivo* absorption by an individual upon ingestion. In view of the great usefulness of HPMCAS, much research has been spent on modifying HPMCAS and processes for preparing them.

International Patent Applications WO 2005/115330 and WO 2011/159626 disclose HPMCAS polymers with a specific combination of substitution levels.

International Patent Application WO 2014/133885 discloses HPMCAS of a specific substitution pattern of succinoyl groups and acetyl groups. The HPMCAS is produced by reacting acetic acid and succinic acid in separate steps with hydroxypropyl methylcellulose.

It is recognized in the art that the substitution levels and the substitution pattern of HPMCAS have an impact on its ability to increase the bioavailability of poorly water-soluble drugs. It is believed that the molecular weight of an esterified cellulose ether like HPMCAS also has an important impact on its ability to increase the bioavailability of poorly water-soluble drugs. Edgar et al., Cellulose (2007), 14:49-64 mention a study on microparticle formation of theophylline with two CABs (cellulose acetate butyrates) of similar composition, differing substantially only in molecular weight. The release of theophylline was slowed dramatically by higher polymer molecular weight, speeded dramatically by lower particle size, and reduced substantially by particle formation from a higher viscosity solution.

International Patent Applications WO2014/031447 and WO2014/031448 disclose methods of controlling the molecular weight of HPMCAS. WO2014/031447 discloses that the molecular weight of HPMCAS increases with decreasing molar ratio [aliphatic carboxylic acid / anhydroglucose units of cellulose ether]. WO2014/031448 discloses that the molecular weight of HPMCAS increases with increasing molar ratio [alkali metal carboxylate / anhydroglucose units of cellulose ether]. The aliphatic carboxylic acid and the alkali metal carboxylate are used as reaction diluent and reaction catalyst, respectively.

In view of the great utility and importance of esterified cellulose ethers like HPMCAS it is an object of the present invention to enrich the art and to find new ways of producing esterified cellulose ethers. It is a preferred object of the present invention to find new ways of producing high molecular weight esterified cellulose ethers.

## SUMMARY

Surprisingly, it has been found that the weight average molecular weight of an ester of a cellulose ether can be increased by changing certain process parameters in the process for esterifying a cellulose ether, even when the types and amounts of reactants, i.e., the cellulose ether and esterifying agents, and the types and amounts of other components, such as diluent and reaction catalyst, are the same as in known processes.

One aspect of the present invention is a process for reacting a cellulose ether with an aliphatic monocarboxylic acid anhydride and a dicarboxylic acid anhydride, wherein the process comprises the steps of

a) preparing a reaction mixture comprising the cellulose ether, the dicarboxylic acid anhydride and a reaction diluent and heating the reaction mixture to a temperature of from 60°C to 110 °C prior to, during or after mixing the components of the reaction mixture, and
b) adding the monocarboxylic acid anhydride continuously or in at least three portions to the reaction mixture of step a) and completing the reaction.

Another aspect of the present invention is a method of producing an esterified cellulose ether of increased weight average molecular weight in a process for reacting a cellulose ether with an aliphatic monocarboxylic acid anhydride and a dicarboxylic acid anhydride, wherein the process comprises the steps of

a) preparing a reaction mixture comprising the cellulose ether, the dicarboxylic acid anhydride and a reaction diluent and heating the reaction mixture to a temperature of from 60°C to 110 °C prior to, during or after mixing the components of the reaction mixture, and
b) producing an esterified cellulose ether of increased weight average molecular weight by adding the monocarboxylic acid anhydride continuously or in at least three portions to the reaction mixture of step a), and completing the reaction.

## DESCRIPTION OF EMBODIMENTS

The cellulose ether used as a starting material in the process of the present invention has a cellulose backbone having β-1,4 glycosidically bound D-glucopyranose repeating units, designated as anhydroglucose units in the context of this invention. The cellulose ether preferably is an alkyl cellulose, hydroxyalkyl cellulose or hydroxyalkyl alkylcellulose. This means that in the cellulose ether utilized in the process of the present invention, at least a part of the hydroxyl groups of the anhydroglucose units are substituted by alkoxyl groups or hydroxyalkoxyl groups or a combination of alkoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the cellulose ether. Preferably a single kind of hydroxyalkoxyl group, more preferably hydroxypropoxyl, is present. The alkoxyl groups are typically methoxyl, ethoxyl and/or propoxyl groups. Methoxyl groups are preferred.

Illustrative of the above-defined cellulose ethers are alkylcelluloses, such as methylcellulose, ethylcellulose, and propylcellulose; hydroxyalkylcelluloses, such as hydroxyethylcellulose, hydroxypropylcellulose, and hydroxybutylcellulose; and hydroxyalkyl alkylcelluloses, such as hydroxyethyl methylcellulose, hydroxymethyl ethylcellulose, ethyl hydroxyethylcellulose, hydroxypropyl methylcellulose, hydroxypropyl ethylcellulose, hydroxybutyl methylcellulose, and hydroxybutyl ethylcellulose; and those having two or more hydroxyalkyl groups, such as hydroxyethylhydroxypropyl methylcellulose. Most preferably, the cellulose ether is a hydroxyalkyl methylcellulose, such as hydroxypropyl methylcellulose.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the cellulose ether. It is to be understood that during the hydroxyalkylation reaction the hydroxyl group of a hydroxyalkoxyl group bound to the cellulose backbone can be further etherified by an alkylation agent, e.g. a methylation agent, and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxy units are covalently bound to each other by ether bonding. Within this definition it is not important whether the terminal hydroxyl group of a hydroxyalkoxyl substituent is further alkylated, e.g. methylated, or not; both alkylated and non-alkylated hydroxyalkoxyl substituents are included for the determination of MS(hydroxyalkoxyl). The cellulose ether utilized in the process of the invention generally has a molar substitution of hydroxyalkoxyl groups in the range 0.05 to 1.00, preferably 0.08 to 0.90, more preferably 0.12 to 0.70, most preferably 0.15 to 0.60, and particularly 0.20 to 0.40.

The average number of hydroxyl groups substituted by alkoxyl groups, such as methoxyl groups, per anhydroglucose unit, is designated as the degree of substitution of alkoxyl groups, DS(alkoxyl). In the above-given definition of DS, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The cellulose ethers utilized in the process of the invention generally have a DS(alkoxyl) in the range of 1.0 to 2.5, preferably from 1.1 to 2.4 , more preferably from 1.2 to 2.2 most preferably from 1.6 to 2.05, and particularly from 1.7 to 2.05.

The degree of substitution of alkoxyl groups and the molar substitution of hydroxyalkoxyl groups can be determined by Zeisel cleavage of the cellulose ether with hydrogen iodide and subsequent quantitative gas chromatographic analysis (G. Bartelmus and R. Ketterer, Z. Anal. Chem., 286 (1977) 161-190). Most preferably the cellulose ether utilized in the process of the invention is hydroxypropyl methylcellulose having a DS(methoxyl) within the ranges indicated above for DS(alkoxyl) and an MS(hydroxypropoxyl) within the ranges indicated above for MS(hydroxyalkoxyl).

The cellulose ether used as a starting material in the process of the present invention generally has a viscosity of up to 200 mPa·s, preferably up to 100 mPa·s, more preferably up to 50 mPa·s, and most preferably up to 5 mPa·s, measured as a 2 weight-% aqueous solution at 20 °C according to ASTM D2363 - 79 (Reapproved 2006). Generally their viscosity is at least 1.2 mPa·s, typically at least 1.8 mPa·s, even more typically at least 2.4 mPa·s, and most typically at least 2.8 mPa·s, measured as a 2 weight-% aqueous solution at 20 °C. Cellulose ethers of such viscosity can be obtained by subjecting a cellulose ether of higher viscosity to a partial depolymerization process. Partial depolymerization processes are well known in the art and described, for example, in European Patent Applications EP 1 141 029; EP 0 210 917; EP 1 423 433; and US Patent No. 4,316,982. Alternatively, partial depolymerization can be achieved during the production of the cellulose ethers, for example by the presence of oxygen or an oxidizing agent.

The molar number of anhydroglucose units of the cellulose ether utilized in the process of the present invention can be determined from the weight of the cellulose ether used as a starting material, by calculating the average molecular weight of the substituted anhydroglucose units from the DS(alkoxyl) and MS(hydroxyalkoxyl).

In step a) of the process of the present invention a reaction mixture is prepared which comprises the cellulose ether, a dicarboxylic acid anhydride and a reaction diluent. A preferred dicarboxylic acid anhydride is succinic anhydride, maleic anhydride or phthalic anhydride. Succinic anhydride or phthalic anhydride is more preferred. Succinic anhydride is the most preferred dicarboxylic acid anhydride. The molar ratio between the anhydride of a dicarboxylic acid and the anhydroglucose units of the cellulose ether generally is at least 0.01 / 1, preferably at least 0.04 / 1 and more preferably at least 0.2 / 1. The molar ratio between the anhydride of a dicarboxylic acid and the anhydroglucose units of cellulose ether generally is up to 2.5 / 1, preferably up to 1.5 / 1, and more preferably up to 1 / 1.

The reaction diluent preferably is an aliphatic carboxylic acid, such as acetic acid, propionic acid, or butyric acid. The reaction diluent can comprise minor amounts of other solvents or diluents which are liquid at room temperature and do not react with the cellulose ether, such as halogenated $C_1$-$C_3$ derivatives, such as dichloro methane, or dichloro methyl ether, but the amount of the aliphatic carboxylic acid generally is more than 50 percent, preferably at least 75 percent, and more preferably at least 90 percent, based on the total weight of the reaction diluent. Most preferably the reaction diluent essentially consists of an aliphatic carboxylic acid. The reaction mixture in step a) generally comprises from 100 to 2,000 parts by weight, preferably from 100 to 1,000 parts by weight, and more preferably from 100 to 250 parts by weight of a reaction diluent per 100 parts by weight of the cellulose ether.

Additionally, the reaction mixture in step a) of the process of the present invention typically comprises an esterification catalyst, preferably an alkali metal carboxylate, such as sodium acetate or potassium acetate. The amount of the alkali metal carboxylate is preferably 20 to 200 parts by weight of the alkali metal carboxylate per 100 parts by weight of the cellulose ether.

The reaction mixture in step a) of the process of the present invention is heated to a temperature of from 60 °C to 110 °C, preferably from 70 to 100 °C prior to, during or after mixing the components of the reaction mixture. In a preferred embodiment the cellulose ether, the reaction diluent and typically the esterification catalyst are first heated to and kept in the mentioned temperature range from 15 to 120 minutes, preferably from 30 to 90 minutes under agitation, followed by addition of the anhydride of a dicarboxylic acid.

In step b) of the process of the present invention an aliphatic monocarboxylic acid anhydride is added continuously or in at least three portions, preferably in at least four portions, more preferably in at least five portions and most preferably in at least 6 portions to the reaction mixture of step a) and the reaction is completed. Preferred aliphatic monocarboxylic acid anhydrides are acetic anhydride, butyric anhydride and propionic anhydride. Preferably the aliphatic monocarboxylic acid anhydride is added continuously or in 4 to 40 portions, more preferably in 5 to 20 portions, even more preferably in 6 to 15 portions, and most preferably in 7 to 10 portions. Preferably the portions of aliphatic monocarboxylic acid anhydride are about of equal size. The aliphatic monocarboxylic acid anhydride is preferably added over a time period of 30 min. to 12 hours, more preferably over a time period of 4 to 10, and most preferably 5 to 8 hours.

The molar ratio between the total amount of anhydride of aliphatic monocarboxylic acid and the anhydroglucose units of the cellulose ether generally is 0.1 / 1 or more, preferably 0.3 / 1 or more, and more preferably 0.5 / 1 or more, most preferably 1.0 / 1 or more, and particularly 2.0 / 1 or more. The molar ratio between the aliphatic monocarboxylic acid anhydride and the anhydroglucose units of the cellulose ether generally is 10 / 1 or less, preferably 8 / 1 or less, more preferably 6 / 1 or less, most preferably 4 / 1 or less, and particularly 3.5 / 1 or less. After addition of the last amount of aliphatic monocarboxylic acid anhydride, the reaction mixture is kept in the range from 60 °C to 110 °C, preferably from 70 to 100 °C for an additional 10 minutes to 12 hours, preferably 30 minutes to 3 hours.

More preferably the cellulose ether is esterified with succinic anhydride or phthalic anhydride in combination with an aliphatic monocarboxylic acid anhydride selected from the group consisting of acetic anhydride, butyric anhydride and propionic anhydride. Most preferably, hydroxypropyl methylcellulose is reacted with succinic anhydride and acetic anhydride to produce hydroxypropyl methyl cellulose acetate succinate.

After completion of the esterification reaction, the reaction product can be precipitated from the reaction mixture in a known manner, for example by contacting the reaction mixture with a large volume of water, such as described in U.S. Patent No. 4,226,981, International Patent Application WO 2005/115330 or European Patent Application EP 0 219 426. In a preferred embodiment of the invention the reaction product is precipitated from the reaction mixture as described in International Patent Application PCT/US13/030394, published as WO2013/148154, to produce an esterified cellulose ether in the form of a powder.

By the process of the present invention preferably esterified cellulose ethers are produced which comprise groups of the formula - C(O) - R - COOH, wherein R is a divalent aliphatic or aromatic hydrocarbon group, such as - C(O) - CH$_2$ - CH$_2$ - COOH, - C(O) - CH = CH - COOH or - C(O) - C$_6$H$_4$ - COOH, and monovalent acyl groups, such as acetyl, propionyl, or butyryl, such as n-butyryl or i-butyryl. Specific examples of esterified cellulose ethers are hydroxypropyl methyl cellulose acetate phthalate (HPMCAP), hydroxypropyl methyl cellulose acetate maleate (HPMCAM) or hydroxypropyl methylcellulose acetate succinate (HPMCAS); hydroxypropyl cellulose acetate succinate (HPCAS), hydroxybutyl methyl cellulose propionate succinate (HBMCPrS), hydroxyethyl hydroxypropyl cellulose propionate succinate (HEHPCPrS); or methyl cellulose acetate succinate (MCAS). Hydroxypropyl methylcellulose acetate succinate (HPMCAS) is the most preferred esterified cellulose ether.

The esterified cellulose ethers produced according to the process of the present invention generally have a degree of substitution of aliphatic monovalent acyl groups, such as acetyl, propionyl, or butyryl groups, of at least 0.05, preferably at least 0.10, and more preferably at least 0.25. The esterified cellulose ethers generally have a degree of substitution of aliphatic monovalent acyl groups of up to 1.5, preferably up to 1.0, and more preferably up to 0.6. The esterified cellulose ethers generally have a degree of substitution of groups of formula -C(O) - R - COOH, such as succinoyl, of at least 0.01, preferably at least 0.05, and most preferably at least 0.10. The esterified cellulose ethers generally have a degree of substitution of groups of formula -C(O) - R - COOH of up to 1.3, preferably up to 0.8, and more preferably up to 0.5.

The total degree of ester substitution is generally at least 0.06, preferably at least 0.10, more preferably at least 0.20, and most preferably at least 0.25. The total degree of ester substitution is generally not more than 1.5, preferably not more than 1.2, more preferably not more than 0.90 and most preferably not more than 0.70.

The content of the acetate and succinate ester groups is determined according to "Hypromellose Acetate Succinate, United States Pharmacopia and National Formulary, NF 29, pp. 1548-1550". Reported values are corrected for volatiles (determined as described in section "loss on drying" in the above HPMCAS monograph). The method may be used in analogue manner to determine the content of propionyl, butyryl, phthalyl and other ester groups. The content of ether groups in the esterified cellulose ether is determined in the same manner as described for "Hypromellose", United States Pharmacopeia and National Formulary, USP 35, pp 3467-3469. The contents of ether and ester groups obtained by the above analyses are converted to DS and MS values of individual substituents according to the formulas below. The formulas may be used in analogue manner to determine the DS and MS of substituents of other cellulose ether esters.

% cellulose backbone

$$= 100 - \left( \%MeO * \frac{M(OCH_3) - M(OH)}{M(OCH_3)} \right)$$
$$- \left( \%HPO * \frac{M(OCH_2CH(OH)CH_3) - M(OH)}{M(OCH_2CH(OH)CH_3)} \right)$$
$$- \left( \%Acetyl * \frac{M(COCH_3) - M(H)}{M(COCH_3)} \right)$$
$$- \left( \%Succinoyl * \frac{M(COC_2H_4COOH) - M(H)}{M(COC_2H_4COOH)} \right)$$

$$DS(Me) = \frac{\frac{\%MeO}{M(OCH_3)}}{\frac{\%cellulose\ backbone}{M(AGU)}} \qquad MS(HP) = \frac{\frac{\%HPO}{M(HPO)}}{\frac{\%cellulose\ backbone}{M(AGU)}}$$

$$DS(Acetyl) = \frac{\frac{\%Acetyl}{M(Acetyl)}}{\frac{\%cellulose\ backbone}{M(AGU)}} \qquad DS(Succinoyl) = \frac{\frac{\%Succinoyl}{M(Succinoyl)}}{\frac{\%cellulose\ backbone}{M(AGU)}}$$

M(MeO) = M(OCH$_3$) = 31.03 Da          M(HPO) = M(OCH$_2$CH(OH)CH$_3$) = 75.09 Da
M(Acetyl) = M(COCH$_3$) = 43.04 Da          M(Succinoyl) = M(COC$_2$H$_4$COOH) = 101.08 Da
M(AGU) = 162.14 Da          M(OH) = 17.008 Da          M(H) = 1.008 Da

By convention, the weight percent is an average weight percentage based on the total weight of the cellulose repeat unit, including all substituents. The content of the methoxyl group is reported based on the mass of the methoxyl group (i.e., -OCH$_3$). The content of the hydroxyalkoxyl group is reported based on the mass of the hydroxyalkoxyl group (i.e., -O-alkylene-OH); such as hydroxypropoxyl (i.e., -O-CH$_2$CH(CH$_3$)-OH). The content of the aliphatic monovalent acyl group is reported based on the mass of -C(O) - R$_1$ wherein R$_1$ is a monovalent aliphatic group, such as acetyl (-C(O)-CH$_3$). The content of the group of formula -C(O) - R - COOH is reported based on the mass of this group, such as the mass of succinoyl groups (i.e., - C(O) - CH$_2$ - CH$_2$ - COOH).

Esterified cellulose ethers are produced by the process of the present invention which have a surprisingly high weight average molecular weight $M_w$. Esterified cellulose ethers of typically up to 500,000 Dalton, more typically up to 450,000 Dalton, and most typically up to 400,000 Dalton are produced by the process of the present invention. Generally they have a weight average molecular weight $M_w$ of at least 100,000 Dalton, preferably at least 150,000 Dalton, more preferably at least 200,000 Dalton, even more preferably at least 250,000 Dalton, and most preferably at least 300,000 Dalton. Surprisingly, it has been found that esterified cellulose ethers having a considerably higher weight average molecular weight $M_w$ are produced according to the process of the present invention than in comparative processes wherein i) neither the dicarboxylic acid anhydride nor the aliphatic monocarboxylic acid anhydride are added continuously or in portions, or ii) the dicarboxylic acid anhydride is added continuously in portions, but not the aliphatic monocarboxylic acid anhydride, or iii) both the dicarboxylic acid anhydride and the aliphatic monocarboxylic acid anhydride are added continuously or in portions to the reaction mixture.

$M_w$ and $M_n$ are measured according to Journal of Pharmaceutical and Biomedical Analysis 56 (2011) 743 using a mixture of 40 parts by volume of acetonitrile and 60 parts by volume of aqueous buffer containing 50 mM NaH$_2$PO$_4$ and 0.1 M NaNO$_3$ as mobile phase. The mobile phase is adjusted to a pH of 8.0. The measurement of $M_w$ and $M_n$ $M_z$ is described in more details in the Examples.

Some embodiments of the invention will now be described in detail in the following Examples.

EXAMPLES

Unless otherwise mentioned, all parts and percentages are by weight. In the Examples the following test procedures are used.

Content of ether and ester groups of Hydroxypropyl Methyl Cellulose Acetate Succinate (HPMCAS)

The content of ether groups in the esterified cellulose ether was determined in the same manner as described for "Hypromellose", United States Pharmacopeia and National Formulary, USP 35, pp 3467-3469.

The ester substitution with acetyl groups (-CO-CH$_3$) and the ester substitution with succinoyl groups (-CO-CH$_2$-CH$_2$-COOH) were determined according to Hypromellose Acetate Succinate, United States Pharmacopia and National Formulary, NF 29, pp. 1548-1550". Reported values for ester substitution were corrected for volatiles (determined as described in section "loss on drying" in the above HPMCAS monograph).

Determination of M$_w$ and M$_n$ of HPMCAS

Mw and Mn were measured according to Journal of Pharmaceutical and Biomedical Analysis 56 (2011) 743 unless stated otherwise. The mobile phase was a mixture of 40 parts by volume of acetonitrile and 60 parts by volume of aqueous buffer containing 50 mM NaH2PO4 and 0.1 M NaNO3. The mobile phase was adjusted to a pH of 8.0. Solutions of the cellulose ether esters were filtered into a HPLC vial through a syringe filter of 0.45 $\mu$m pore size.

More specifically, the utilized Chemicals and solvents were:
Polyethylene oxide standard materials (abbreviated as PEOX 20 K and PEOX 30 K) were purchased from Agilent Technologies, Inc. Palo Alto, CA, catalog number PL2083-1005 and PL2083-2005.

Acetonitrile (HPLC grade ≥ 99.9 %, CHROMASOL plus), catalog number 34998, sodium hydroxide (semiconductor grade, 99.99 %, trace metal base), catalog number 306576, water (HPLC grade, CHROMASOLV Plus) catalog number 34877 and sodium nitrate (99,995 %, trace metal base) catalog number 229938 were purchased from Sigma-Aldrich, Switzerland.

Sodium dihydrogen phosphate (≥ 99.999 % TraceSelect) catalog number 71492 was purchased from FLUKA, Switzerland.

The normalization solution of PEOX20 K at 5 mg/mL, the standard solution of PEOX30 K at 2 mg/mL, and the sample solution of HPMCAS at 2 mg/mL were prepared by adding a weighed amount of polymer into a vial and dissolving it with a measured volume of mobile phase. All solutions were allowed to dissolve at room temperature in the capped vial for 24 h with stirring using a PTFE-coated magnetic stirring bar.

The normalization solution (PEOX 20k, single preparation, N) and the standard solution (PEOX30 K, double preparation, S1 and S2) were filtered into a HPLC vial through a syringe filter of 0.02 $\mu$m pore size and 25 mm diameter (Whatman Anatop 25, catalog number 6809-2002), Whatman.

The test sample solution (HPMCAS, prepared in duplicate, T1, T2) and a laboratory standard (HPMCAS, single preparation, LS) were filtered into a HPLC vial through a syringe filter of 0.45 $\mu$m pore size (Nylon, e.g. Acrodisc 13 mm VWR catalog number 514-4010).

Chromatographic condition and run sequence were conducted as described by Chen, R. et al.; Journal of Pharmaceutical and Biomedical Analysis 56 (2011) 743- 748). The SEC-MALLS instrument set-up included a HP1100 HPLC system from Agilent Technologies, Inc. Palo Alto, CA; a DAWN Heleos II 18 angle laser light scattering detector and a OPTILAB rex refractive index detector, both from Wyatt Technologies, Inc. Santa Barbara, CA. The analytical size exclusion column (TSK-GEL® GMPWXL, 300 × 7.8 mm) was purchased from Tosoh Bioscience. Both the OPTILAB and the DAWN were operated at 35 °C. The analytical SEC column was operated at room temperature (24 ± 5 °C). The mobile phase was a mixture of 40 volume parts of acetonitrile and 60 volume parts of aqueous buffer containing 50 mM NaH2PO4 and 0.1 M NaNO3 prepared as follows:
Aqueous buffer: 7.20 g of sodium dihydrogen phosphate and 10.2 g of sodium nitrate were added to 1.2 L purified water in a clean 2 L glass bottle under stirring until dissolution.

Mobile phase: 800 mL of acetonitrile were added to 1.2 L of the aqueous buffer prepared above, and stirred until a good mixture was achieved and the temperature equilibrated to ambient temperature.

The mobile phase was pH adjusted to 8.0 with 10M NaOH and filtered through a 0.2 m nylon membrane filter. The flow rate was 0.5 mL/min with in-line degassing. The injection volume was 100 $\mu$L and the analysis time was 35 min.

The MALLS data were collected and processed by Wyatt ASTRA software (version 5.3.4.20) using dn/dc value (refractive index increment) of 0.120 mL/g for HPMCAS. The light scattering signals of detector Nos. 1-4, 17, and 18) were not used in the molecular weight calculation. A representative chromatographic run sequence is given below: B, N, LS, S1 (5x), S2, T1 (2x), T2 (2x), T3 (2x), T4 (2x), S2, T5(2x), etc., S2, LS, W, where, B represents blank injection of mobile phase, N1 represents normalization solution; LS represents a laboratory standard HPMCAS; S1 and S2 represent standard solutions one and two, respectively; T1, T2, T3, T4, and T5 represent test sample solutions and W represents water injection. (2x) and (5x) denote the number of injections of the same solution.

Both the OPTILAB and the DAWN were calibrated periodically according to the manufacturer's recommended procedures and frequency. A 100 $\mu$L injection of a 5 mg/mL polyethylene oxide standard (PEOX20 K) was employed for normalizing all angle light scattering detectors relative to 90° detector for each run sequence.

Use of this mono-dispersed polymer standard also enabled the volume delay between the OPTILAB and the DAWN to be determined, permitting proper alignment of the light scattering signals to the refractive index signal. This is necessary for the calculation of the weight-averaged molecular weight (Mw) for each data slice.

## Production of HPMCAS of Example 1

A hydroxypropyl methylcellulose (HPMC), glacial acetic acid and sodium acetate were introduced in the amounts listed in Table 1 below into a reaction vessel. The amount of HPMC was calculated on a dried basis. The HPMC had a methoxyl substitution ($DS_M$) and a hydroxypropoxyl substitution ($MS_{HP}$) as listed in Table 2 below and a viscosity of about 3 mPa·s, measured as a 2 weight-% aqueous solution at 20 °C according to ASTM D2363 - 79 (Reapproved 2006). The HPMC is commercially available from The Dow Chemical Company as Methocel E3 LV Premium cellulose ether. The sodium acetate and the HPMC were dissolved in the acetic acid at 85°C for one hour under stirring. Afterwards the whole amount of succinic anhydride, as listed in Table 1 below, was quickly added to the reactor. Immediately thereafter, 37.78 g of acetic anhydride was added to the reactor. Each time after 20 min; 40 min; 60 min; 80 min; and 100 min additional 37.78 g of acetic anhydride was added to the reactor. After 120 min. 26.8 g of acetic anhydride was added to the reactor. The total reaction time at 85°C was 5 hours, calculated from the addition of the first portion of acetic anhydride. The product was removed from the reactor, precipitated in 1.8 L of water and washed with water having a temperature of 21°C by applying high shear mixing using an Ultra-Turrax stirrer S50-G45 running at 5200 rpm. Washing was conducted in several portions with intermediate filtration steps to obtain HPMCAS of high purity. After the last filtration step the product was dried at 50°C overnight.

## Production of HPMCAS of Comparative Example A

The same HPMC as in Example 1, glacial acetic acid and sodium acetate were introduced in the amounts listed in Table 1 below into a reaction vessel. The sodium acetate and the HPMC were dissolved in the acetic acid at 85°C for one hour under stirring. Afterwards the whole amounts of succinic anhydride and acetic anhydride, as listed in Table 1 below, were quickly added to the reactor. After allowing the reaction mixture to react for 5 hours at 85°C, calculated from the addition of succinic anhydride and acetic anhydride, 1.8 L of water was added to the reactor under stirring to precipitate the HPMCAS. The precipitated product was removed from the reactor and washed with water having a temperature of 21°C by applying high shear mixing using an Ultra-Turrax stirrer S50-G45 running at 5200 rpm. Washing was conducted in several portions with intermediate filtration steps to obtain HPMCAS of high purity. After the last filtration step the product was dried at 50°C overnight.

## Production of HPMCAS of Comparative Example B (published as Comparative example D in WO 2014/137777)

Glacial acetic acid, acetic anhydride, the same HPMC as in Example 1, succinic anhydride and sodium acetate (water free) were introduced in the amounts listed in Table 1 below into a reaction vessel under thorough stirring to produce a homogeneous reaction mixture. The mixture was heated at 85° C with agitation for 3.5 hours, to effect esterification. 1.8 L of water was added to the reactor under stirring to precipitate the HPMCAS. The precipitated product was removed from the reactor, washed and dried.

## Production of HPMCAS of Comparative Example C

The same HPMC as in Example 1, glacial acetic acid and sodium acetate were introduced in the amounts listed in Table 1 below into a reaction vessel. The sodium acetate and the HPMC were dissolved in the acetic acid at 85°C for one hour under stirring. Afterwards the whole amount of acetic anhydride, as listed in Table 1 below, was quickly added to the reactor. Then 8.26 g of succinic anhydride was added to the reactor. Each time after 20 min; 40 min; 60 min; 80 min; and 100 min additional 8.26 g of succinic anhydride was added to the reactor. After 120 min. 5.0 g of succinic anhydride was added to the reactor. The total reaction time at 85°C was 5 hours, calculated from the addition of the first portion of succinic anhydride. Then 1.8 L of water was added to the reactor under stirring to precipitate the HPMCAS. The precipitated product was removed from the reactor and washed with water having a temperature of 21 °C by applying high shear mixing using an Ultra-Turrax stirrer S50-G45 running at 5200 rpm. Washing was conducted in several portions with intermediate filtration steps to obtain HPMCAS of high purity. After the last filtration step the product was dried at 50°C overnight.

## Production of HPMCAS of Comparative Example D

The same HPMC as in Example 1, glacial acetic acid and sodium acetate were introduced in the amounts listed in Table 1 below into a reaction vessel. The sodium acetate and the HPMC were dissolved in the acetic acid at 85°C for one hour under stirring. Then 37.78 g of acetic anhydride and 8.26 g of succinic anhydride were added to the reactor. Each time after 20 min; 40 min; 60 min; 80 min; and 100 min 37.78 g of acetic anhydride and 8.26 g of succinic anhydride were added to the reactor. After 120 min. 26.8 g of acetic anhydride and 5.0 g of succinic anhydride were added to the reactor. The total reaction time at 85°C was 5 hours, calculated from the addition of the first portion of acetic anhydride and succinic anhydride. Then 1.8 L of water was added to the reactor under stirring to precipitate the HPMCAS. The precipitated product was removed from the reactor and washed with water having a temperature of 21 °C by applying high shear mixing using an Ultra-Turrax stirrer S50-G45 running at 5200 rpm. Washing was conducted in several portions with intermediate filtration steps to obtain HPMCAS of high purity. After the last filtration step the product was dried at 50°C overnight.

The properties of the HPMCAS of Example 1 and Comparative Examples A - D are listed in Table 2 below. In Table 2 the abbreviations have the following meanings:

$DS_M$= DS(methoxyl): degree of substitution with methoxyl groups;
$MS_{HP}$ = MS(hydroxypropoxyl): molar substitution with hydroxypropoxyl groups;
$DS_{Ac}$: degree of substitution with acetyl groups;
$DS_s$: degree of substitution with succinoyl groups;
ac. anh.: acetic anhydride;
succ. anh.: succinic anhydride.

The results in Table 2 below illustrate that in Example 1, where acetic anhydride is added in portions, a much higher weight average molecular weight $M_w$ of the produced HPMCAS is achieved than when i) neither acetic anhydride nor succinic anhydride are added in portions (as in Comparative Examples A and B) or ii) only succinic anhydride is added in portions (as in Comparative Example C) or iii) acetic anhydride and succinic anhydride are added in portions (as in Comparative Example D).

Table 1

| (Comp.) Example | HPMC | | acetic acid | | Succinic anhydride | | Acetic anhydride | | Sodium acetate | | Reaction time (h) | Reaction temp. (°C) | addition in portions |
|---|---|---|---|---|---|---|---|---|---|---|---|---|---|
| | g | mol | g | mol/mol HPMC | g | mol/mol HPMC | g | mol/mol HPMC | g | mol/mol HPMC | | | |
| 1 | 195 | 0.96 | 442 | 7.6 | 54.6 | 0.57 | 253.5 | 2.69 | 195.0 | 2.47 | 5 | 85 | acetic anhydride |
| A | 195 | 0.96 | 442 | 7.6 | 54.6 | 0.57 | 253.5 | 2.69 | 195.0 | 2.47 | 3.5 | 85 | none |
| B | 195 | 0.96 | 442 | 7.6 | 54.6 | 0.57 | 253.5 | 2.69 | 195.0 | 2.47 | 5 | 85 | none |
| C* | 195 | 0.96 | 442 | 7.6 | 54.6 | 0.57 | 253.5 | 2.69 | 195.0 | 2.47 | 5 | 85 | succinic anhydride |
| D* | 195 | 0.96 | 442 | 7.6 | 54.6 | 0.57 | 253.5 | 2.69 | 195.0 | 2.47 | 5 | 85 | ac. anh. + succ. anh. |

Table 2

| (Comparative) Example | Molecular weight (kDA) | | Methoxyl (%) | Hydroxy-propoxyl (%) | Acetyl (%) | Succinoyl (%) | $DS_M$ | $MS_{HP}$ | $DS_{Ac}$ | $DS_s$ |
|---|---|---|---|---|---|---|---|---|---|---|
| | Mn | Mw | | | | | | | | |
| 1 | 119 | 370 | 22.0 | 6.8 | 7.9 | 16.5 | 1.89 | 0.24 | 0.49 | 0.44 |
| A | 43 | 130 | 23.0 | 7.1 | 10.8 | 11.3 | 1.93 | 0.25 | 0.65 | 0.29 |
| B | 36 | 139 | 22.7 | 7.5 | 11.0 | 12.1 | 1.94 | 0.26 | 0.68 | 0.32 |
| C* | 20 | 56 | 23.7 | 7.3 | 13.6 | 6.1 | 1.93 | 0.25 | 0.80 | 0.15 |
| D* | 55 | 173 | 23.0 | 7.1 | 10.6 | 11.2 | 1.92 | 0.24 | 0.64 | 0.29 |
| * Comparative, but not prior art | | | | | | | | | | |

**Claims**

1. A process for reacting a cellulose ether with an aliphatic monocarboxylic acid anhydride and a dicarboxylic acid anhydride, wherein the process comprises the steps of

   a) preparing a reaction mixture comprising the cellulose ether, the dicarboxylic acid anhydride and a reaction diluent and heating the reaction mixture to a temperature of from 60°C to 110 °C prior to, during or after mixing the components of the reaction mixture, wherein the reaction diluent is an aliphatic carboxylic acid and wherein reaction mixture additionally comprises an esterification catalyst which is an alkali metal carboxylate, and
   b) adding the monocarboxylic acid anhydride continuously or in at least three portions to the reaction mixture of step a) and completing the reaction,
   wherein the cellulose ether is esterified with (i) succinic anhydride or phthalic anhydride and (ii) an aliphatic monocarboxylic acid anhydride selected from the group consisting of acetic anhydride, butyric anhydride and propionic anhydride and wherein the cellulose ether is hydroxypropyl methylcellulose.

2. The process of claim 1 wherein the monocarboxylic acid anhydride is added in at least four portions at different reactions times to the reaction mixture of step a).

3. The process of claim 1 or claim 2 wherein the cellulose ether has a viscosity of from 1.2 to 200 mPa·s, measured as a 2 weight-% solution in water at 20 °C according to ASTM D2363 - 79, reapproved 2006.

4. The process of claim 1 wherein hydroxypropyl methylcellulose is esterified with succinic anhydride and acetic anhydride to produce hydroxypropyl methyl cellulose acetate succinate.

5. The process of any one of claims 1 to 4 wherein the produced esterified cellulose ether has a weight average molecular weight $M_w$ of from 100,000 to 500,000 Dalton.

6. The process of any one of claims 1 to 5 wherein the molar ratio between the anhydride of the aliphatic monocarboxylic acid and the anhydroglucose units of the cellulose ether is from 0.3 / 1 to 4 / 1.

7. The process of any one of claims 1 to 6 wherein the molar ratio between the anhydride of a dicarboxylic acid and the anhydroglucose units of cellulose ether is from 0.04/ 1 to 1.5 / 1.

8. A method of producing an esterified cellulose ether of increased weight average molecular weight in a process for reacting a cellulose ether with an aliphatic monocarboxylic acid anhydride and a dicarboxylic acid anhydride, wherein the process comprises the steps of

   a) preparing a reaction mixture comprising the cellulose ether, the dicarboxylic acid anhydride, a reaction diluent and an esterification catalyst, and heating the reaction mixture to a temperature of from 60°C to 110 °C prior to, during or after mixing the components of the reaction mixture, wherein the reaction diluent is an aliphatic carboxylic acid and the esterification catalyst is an alkali metal carboxylate, and
   b) producing an esterified cellulose ether of increased weight average molecular weight by adding the monocarboxylic acid anhydride continuously or in at least three portions to the reaction mixture of step a), and completing the reaction,
   wherein the cellulose ether is esterified with (i) succinic anhydride or phthalic anhydride and (ii) an aliphatic monocarboxylic acid anhydride selected from the group consisting of acetic anhydride, butyric anhydride and propionic anhydride and wherein the cellulose ether is hydroxypropyl methylcellulose.

**Patentansprüche**

1. Ein Verfahren zur Umsetzung eines Celluloseethers mit einem aliphatischen Monocarbonsäureanhydrid und einem Dicarbonsäureanhydrid, wobei das Verfahren die Schritte des

   a) Herstellens einer Reaktionsmischung umfassend den Celluloseether, das Dicarbonsäureanhydrid und einen Reaktionsverdünner und Erwärmens der Reaktionsmischung auf eine Temperatur von 60 °C bis 110 °C vor, während oder nach dem Vermischen der Bestandteile der Reaktionsmischung, wobei der Reaktionsverdünner eine aliphatische Carbonsäure ist und wobei die Reaktionsmischung zusätzlich einen Veresterungskatalysator umfasst, der ein Alkalimetallcarbonsäuresalz ist und

b) Hinzufügens des Monocarbonsäureanhydrids zu der Reaktionsmischung des Schritts a) in kontinuierlicher Weise oder in mindestens drei Teilen und Vervollständigens der Reaktion,

umfasst, wobei der Celluloseether mit (i) Bernsteinsäureanhydrid oder Phthalsäureanhydrid und (ii) einem aliphatischen Monocarbonsäureanhydrid ausgewählt aus der Gruppe bestehend aus Essigsäureanhydrid, Buttersäureanhydrid und Propionsäureanhydrid verestert wird und wobei der Celluloseether Hydroxypropylmethylcellulose ist.

2. Das Verfahren gemäß Anspruch 1, wobei das Monocarbonsäureanhydrid in mindestens vier Teilen zu unterschiedlichen Reaktionszeiten zu der Reaktionsmischung des Schritts a) hinzugegeben wird.

3. Das Verfahren gemäß Anspruch 1 oder 2, wobei der Celluloseether eine Viskosität von 1,2 bis 200 mPa·s, gemessen als eine 2 gew.-%ige Lösung in Wasser bei 20 °C gemäß ASTM D2363-79, erneut genehmigt 2006, aufweist.

4. Das Verfahren gemäß Anspruch 1, wobei Hydroxypropylmethylcellulose mit Bernsteinsäureanhydrid und Essigsäureanhydrid verestert wird, um Hydroxypropylmethylcelluloseacetatsuccinat herzustellen.

5. Das Verfahren gemäß irgendeinem der Ansprüche 1 bis 4, wobei der hergestellte veresterte Celluloseether ein gewichtsmittleres Molekulargewicht $M_w$ von 100.000 bis 500.000 Dalton aufweist.

6. Das Verfahren gemäß irgendeinem der Ansprüche 1 bis 5, wobei das molare Verhältnis zwischen dem Anhydrid der aliphatischen Monocarbonsäure und den Anhydroglukoseeinheiten des Celluloseethers von 0,3/1 bis 4/1 beträgt.

7. Das Verfahren gemäß irgendeinem der Ansprüche 1 bis 6, wobei das molare Verhältnis zwischen dem Anhydrid einer Dicarbonsäure und den Anhydroglukoseeinheiten des Celluloseethers von 0,04/1 bis 1,5/1 beträgt.

8. Ein Verfahren zur Herstellung eines veresterten Celluloseethers von erhöhtem gewichtsmittlerem Molekulargewicht in einem Verfahren zur Umsetzung eines Celluloseethers mit einem aliphatischen Monocarbonsäureanhydrid und einem Dicarbonsäureanhydrid, wobei das Verfahren die Schritte des

a) Herstellens einer Reaktionsmischung umfassend den Celluloseether, das Dicarbonsäureanhydrid, einen Reaktionsverdünner und einen Veresterungskatalysator und Erwärmens der Reaktionsmischung auf eine Temperatur von 60 °C bis 110 °C vor, während oder nach dem Vermischen der Bestandteile der Reaktionsmischung, wobei der Reaktionsverdünner eine aliphatische Carbonsäure ist und der Veresterungskatalysator ein Alkalimetallcarbonsäuresalz ist und

b) Herstellens eines veresterten Celluloseethers von erhöhtem gewichtsmittlerem Molekulargewicht durch Hinzufügen des Monocarbonsäureanhydrids zu der Reaktionsmischung des Schritts a) in kontinuierlicher Weise oder in mindestens drei Teilen und Vervollständigen der Reaktion,

umfasst, wobei der Celluloseether mit (i) Bernsteinsäureanhydrid oder Phthalsäureanhydrid und (ii) einem aliphatischen Monocarbonsäureanhydrid ausgewählt aus der Gruppe bestehend aus Essigsäureanhydrid, Buttersäureanhydrid und Propionsäureanhydrid verestert wird und wobei der Celluloseether Hydroxypropylmethylcellulose ist.

## Revendications

1. Procédé pour faire réagir un éther de cellulose avec un anhydride d'acide monocarboxylique aliphatique et un anhydride d'acide dicarboxylique, lequel procédé comporte les étapes consistant à

(a) préparer un mélange réactionnel comprenant l'éther de cellulose, l'anhydride d'acide dicarboxylique et un diluant réactionnel, et faire chauffer le mélange réactionnel à une température valant de 60 °C à 110 °C avant, pendant ou après avoir combiné les constituants du mélange réactionnel, le diluant réactionnel étant un acide carboxylique aliphatique et le mélange réactionnel comprenant en plus un catalyseur d'estérification qui est un carboxylate de métal alcalin,

(b) et introduire l'anhydride d'acide monocarboxylique, en continu ou en au moins trois fractions, dans le mélange réactionnel issu de l'étape (a), et mener la réaction à son terme,

étant entendu que l'on estérifie l'éther de cellulose avec (i) de l'anhydride succinique ou de l'anhydride phtalique, et (ii) un anhydride d'acide monocarboxylique aliphatique choisi dans l'ensemble constitué par de l'anhydride acétique, de l'anhydride butyrique et de l'anhydride propionique, et que l'éther de cellulose est de l'hydroxypropyl-méthylcellulose.

2. Procédé selon la revendication 1, dans lequel on introduit l'anhydride d'acide monocarboxylique en au moins quatre fractions à des instants de réaction différents, dans le mélange réactionnel issu de l'étape (a).

3. Procédé selon la revendication 1 ou la revendication 2, dans lequel l'éther de cellulose présente une viscosité comprise dans l'intervalle allant de 1,2 à 200 mPa·s, la viscosité étant déterminée sur une solution à 2 % en poids dans l'eau à 20 °C, conformément à la norme ASTM D2362-79, agréée à nouveau en 2006.

4. Procédé selon la revendication 1, dans lequel on estérifie l'hydroxypropyl-méthylcellulose avec de l'anhydride succinique et de l'anhydride acétique, de manière à produire de l'acétate-succinate d'hydroxypropyl-méthylcellulose.

5. Procédé selon n'importe laquelle des revendications 1 à 4, dans lequel l'éther de cellulose estérifié ainsi produit présente une masse moléculaire moyenne en poids, Mp, comprise dans l'intervalle allant de 100 000 à 500 000 Da (daltons).

6. Procédé selon n'importe laquelle des revendications 1 à 5, dans lequel le rapport molaire entre l'anhydride d'acide monocarboxylique aliphatique et les motifs anhydroglucose de l'éther de cellulose est compris dans l'intervalle allant de 0,3/1 à 4/1.

7. Procédé selon n'importe laquelle des revendications 1 à 6, dans lequel le rapport molaire entre l'anhydride d'acide dicarboxylique et les motifs anhydroglucose de l'éther de cellulose est compris dans l'intervalle allant de 0,04/1 à 1,5/1.

8. Technique de production d'un éther de cellulose estérifié de masse moléculaire moyenne en poids augmentée, au cours d'un procédé pour faire réagir un éther de cellulose avec un anhydride d'acide monocarboxylique aliphatique et un anhydride d'acide dicarboxylique, lequel procédé comporte les étapes consistant à

(a) préparer un mélange réactionnel comprenant l'éther de cellulose, l'anhydride d'acide dicarboxylique, un diluant réactionnel et un catalyseur d'estérification, et faire chauffer le mélange réactionnel à une température valant de 60 °C à 110 °C avant, pendant ou après avoir combiné les constituants du mélange réactionnel, le diluant réactionnel étant un acide carboxylique aliphatique et le catalyseur d'estérification étant un carboxylate de métal alcalin,
(b) et produire un éther de cellulose estérifié de masse moléculaire moyenne en poids augmentée, en introduisant l'anhydride d'acide monocarboxylique, en continu ou en au moins trois fractions, dans le mélange réactionnel issu de l'étape (a), et en menant la réaction à son terme,

étant entendu que l'on estérifie l'éther de cellulose avec (i) de l'anhydride succinique ou de l'anhydride phtalique, et (ii) un anhydride d'acide monocarboxylique aliphatique choisi dans l'ensemble constitué par de l'anhydride acétique, de l'anhydride butyrique et de l'anhydride propionique, et que l'éther de cellulose est de l'hydroxypropyl-méthylcellulose.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4226981 A
- US 4365060 A
- EP 0219426 A
- WO 2005115330 A
- WO 2011159626 A
- WO 2014133885 A
- WO 2014031447 A
- WO 2014031448 A
- EP 1141029 A
- EP 0210917 A
- EP 1423433 A
- US 4316982 A
- US 13030394 W
- WO 2013148154 A

**Non-patent literature cited in the description**

- **EDGAR et al.** *Cellulose,* 2007, vol. 14, 49-64
- **G. BARTELMUS AND R ; KETTERER, Z.** *Anal. Chem.,* 1977, vol. 286, 161-190
- United States Pharmacopeia and National Formulary. *Hypromellose,* 3467-3469
- *Journal of Pharmaceutical and Biomedical Analysis,* 2011, vol. 56, 743
- Hypromellose Acetate Succinate. United States Pharmacopia, vol. 29, 1548-1550
- **CHEN, R. et al.** *Journal of Pharmaceutical and Biomedical Analysis,* 2011, vol. 56, 743-748